# EUROPÄISCHE PATENTSCHRIFT

(11) **EP 0 305 757 B1**
(45) Veröffentlichungstag und Bekanntmachung des Hinweises auf die Patenterteilung: **22.12.1993**
(21) Anmeldenummer: 88112631.2
(22) Anmeldetag: 03.08.1988
(51) Int. Cl.: A61L 15/58, A61L 15/44

(54) **Verfahren zur Herstellung einer Vorrichtung zur gesteuerten Abgabe von Nicotin, und ihre Verwendung.**
Process for the preparation of a device for the controlled release of nicotine, and its use.
Procédé de préparation d'un dispositif de diffusion controlée de la nicotine, et son utilisation.

(30) Priorität: 01.09.1987 DE 3729165; 23.12.1987 DE 3743947
(43) Veröffentlichungstag der Anmeldung: 08.03.1989
(73) Patentinhaber: LTS Lohmann Therapie-Systeme GmbH & Co. KG, 56513 Neuwied (DE)
(72) Erfinder: Jaeger, Halvor, D-7910 Neu-Ulm (DE); Hoffmann, Hans-Rainer, D-5450 Neuwied 22 (DE); Meconi, Reinhold, D-5451 Neuwied 11 (DE); Klein, Robert-Peter, D-5450 Neuwied 11 (DE)
(74) Vertreter: Flaccus, Rolf-Dieter, Dr.

(56) Entgegenhaltungen:
- EP-A- 0 086 468
- EP-A- 0 127 282
- EP-A- 0 144 486
- EP-A- 0 186 019
- WO-A-88/01516
- US-A- 4 515 909
- US-A- 4 597 961

## Beschreibung

Die Erfindung betrifft ein verfahren zur Herstellung einer Vorrichtung zur gesteuerten Abgabe von Nicotin mit einem haftklebenden Nicotinreservoir sowie deren Verwendung.

Nicotinhaltige Pflaster, insbesondere zur Raucherentwöhnung, sind bereits bekannt. So wurde beispielsweise in der DE-OS 34 38 284 (Tilly) ein nicotinhaltiges Depotpflaster beschrieben. In "Drug and Alcohol Dependence, Band 13 (1984) Seiten 2O9-213 wurde von JE.Rose, N.E.Jarvic und K.D. Rose vorgeschlagen, Nicotin transdermal nicotinabhängigen Patienten zuzuführen, um gewohnheitsmäßige Raucher, die nicotinabhängig sind, derart am Inhalieren von carzerogenen Stoffen zu hindern. Es wurden Versuche mit wässriger Nicotinlösung, die mittels einer dünnen Polyethylenschicht gegen Verdampfung nach Aufbringen auf die Haut geschützt wurde, durchgeführt und gefunden, daß Nicotin die Haut permeiert und mittels transdermaler Nicotingaben das gleiche Nicotinniveau, wie durch Rauchen, erzielt werden kann.

Von Etscorn wird in der US-A- 3 597 961 ein einfaches Nicotinpflaster zur Raucherentwöhnung vorgeschlagen, bei dem Nicotin, das in einem Hohlraum eines Pflasters vorliegt, ggf. durch eine nicotindurchlässige Membran überdeckt, mit der Haut in Kontakt gebracht werden kann, um das Permeieren des Nicotins in den menschlichen Körper zu ermöglichen und die Nicotinabhängigkeit von Rauchern zu bekämpfen.

In der DE-OS 36 29 3O4 wird vorgeschlagen, ein Nicotinpflaster unter Vorsehen von Nicotindepots in einer nicotinverteilenden, aus einer Acrylatlösung unter Verdampfung des Lösemittels hergestellten Acrylatmatrix herzustellen.

Nicotinpflaster sind grundsätzlich schwierig zu produzieren, da das Nicotin sehr flüchtig und zudem toxisch ist. Die Herstellung von Nicotinpflasterbestandteilen, insbesondere den Bestandteilen der haftklebenden Matrix aus der Lösung, ist aus mehreren Gründen nachteilig. Sie bedingt einen hohen technischen Aufwand und Kosten für die Handhabung der Lösemittel, außerdem müssen für medizinische Zwecke hochreine und damit teuere Lösemittel eingesetzt werden, um für die Auflösung der Kleber bzw. deren Ausgangsmaterialien eine entsprechende Rückstandsfreiheit in der Vorrichtung sicherzustellen. Ein weiteres Problem besteht darin, Lösemittelfreiheit in der Vorrichtung zu erreichen, wofür teuere Trocknungsstrecken und Absauganlagen erforderlich sind. Bei der Verarbeitung von Nicotin tritt insbesondere das Problem auf, daß beim Abdampfen der Lösemittel ein erheblicher Anteil des leichtflüchtigen Nicotins verdampfen kann, wobei dies wegen der Flüchtigkeit und Giftigkeit des Nicotins äußerst unerwünscht ist.

Es treten also Kosten durch die Verwendung, Wiedergewinnung oder Abscheidung von Lösemitteln und Nicotin auf, um Umweltbelastungen zu vermeiden. Daneben stellt die Brennbarkeit der Lösemittel ein zusätzliches Risiko dar. Es müssen aufwendige Schutzmaßnahmen für die im Betrieb tätigen Personen getroffen werden.

Es ist somit Aufgabe der Erfindung, die oben aufgeführten Nachteile der gattungsgemäßen Verfahren nach dem Stand der Technik zu vermeiden.

Die Aufgabe wird erfindungsgemäß durch ein Verfahren zur Herstellung einer Vorrichtung zur gesteuerten Abgabe von Nicotin mit einem haftklebenden Nicotinreservoir, gelöst, mit Herstellung einer Mischung aus geschmolzenem Haftschmelzkleber mit einer Verarbeitungstemperatur von 40 bis 80°C und Nicotin und Aufbringen der Mischung auf eine Unterlage.

Durch diese Maßnahmen kann erfindungsgemäß eine nicotinhaltige Vorrichtung bei niedrigen Temperaturen ohne Lösemittel unter erheblicher Ersparnis an Materialien schnell ohne die zeitaufwendigen Trocknungsschritte sowie unter geringerer Umweltbelastung hergestellt werden, was unter anderem zu einem erheblich kostengünstigerem Produkt führt.

Nach dem erfindungsgemäßen Verfahren hergestellte Vorrichtungen lassen sich beispielsweise in der Human- oder Tiermedizin, insbesondere zur Raucherentwöhnung einsetzen. Es wurde aber auch bereits vorgeschlagen, das fungizide Nicotin in Pflasterform lokal zur Bekämpfung von Pilzbefall auf der Haut einzusetzen. Sie kann auch als Vorrichtung zur Abgabe des Nicotins als Atem-, Kontakt- oder Fraßgift in der Schädlingsbekämpfung eingesetzt werden.

Vorteilhafte Weiterbildungen der Erfindung ergeben sich aus den Unteransprüchen.

Dabei wird hier unter Haftschmelzkleber jeder Haftklebstoff verstanden, der heiß hinreichend flüssig ist, um problemlos bei Temperaturen oberhalb 4O° C aufgetragen zu werden.

Als erfindungsgemäß einsetzbare Haftschmelzkleber können unter anderem solche eingesetzt werden, die dem Fachmann geläufig sind und wie sie u. a. in den DE-OS 15 94 268 (SUN OIL CO.), DE-OS 24 13 979 (E.I.DU PONT DE NEMOURS),DE-OS 24 35 863 (DYNAMIT NOBEL AG); DE-OS 28 00 302 (CIBA GEIGY); den EP-A- 104 005 (PERSONAL PRODUCTS CO), den JP 6104 2583 und JP 61 281 810, der EP-OS 131 460 (EXXON) und der EP-OS 234 856 (EXXON), der EP-OS 185 992 (EASTMAN KODAK) sowie der US-PS 36 99 963 und der US-PS 4,358,557 (EASTMAN KODAK) herleitbar sind, wobei zur Vermeidung von Wiederholungen auf diesen Stand der Technik ausdrücklich bezug genommen wird.

Als Grundpolymere können bspw. Polyamide, Polyester, Polycaprolactame, Polycaprolacton, Ethylen-Vinylacetat-Copolymere (EVA), Ethylen-Ethylacrylat-Copolymere (EEA), Polyvinylether, Polyacrylatester, Polyvinylacetale, Polyvinylacetate, Styrol-Butadien-Blockpolymere, Isopren-Blockpolymere, Polyurethane, Ethylcellulose, Celluloseacetat-Butyrat, Synthesekautschuke (z.B. Neopren-Kautschuk), Polyisobutylen, Butylgummi, Acrylnitril-Butadien-Mischpolymerisate, Epoxidharze, Melaminharze, Phenol-Formaldehyd-Harze und Resorcin-Formaldehyd-Harze verwendet werden, wobei auch unter anderem die nachfolgend genannten modifizierenden Harze eingesetzt werden können: hydriertes Kolophonium, polymerisiertes Kolophonium, dimerisierte Harzsäuren, disproportioniertes Kolophonium, Methylester von Kolophonium, Glycerinester von hydriertem Kolophonium, Methylester von hydriertem Kolophonium, Pentalester, Triethylenglykolester von hydriertem Kolophonium, Hydroabiethylalkohol und dessen Derivate, Glycerinester, Di-Triolester und Pentaester von Harzsäuren, Pentalester von polymerisiertem Kolophonium, Pentalester von dimerisiertem Kolophonium, Glycerinester von dimerisiertem Kolophonium, Ester von Maleinsäure- oder Phenol-modifiziertem Kolophonium, aromatische und aliphatische Kohlenwasserstoffharze, hydrierte Harze, Polyterpenharze modifizierte Terpenharze, Wachse, niedermolekulares Polyethylen und Polypropylen, Alkyl-Styrol-Copolymere. Diesen Harzen können ggf. Weichmacher, wie z.B. Adipinsäureester, Phosphorsäureester, Phthalsäureester, Polyester, Fettsäureester, Citronensäureester oder Epoxidweichmacher zugesetzt werden. Außerdem können auch Stabilisatioren, wie Tocopherol, substituierte Phenole, Hydrochinone, Brenzkatechine, aromatische Amine, und ggf. auch noch Füllstoffe, wie z. B. Titandioxid, Magnesiumoxid, Zinkoxid und Siliciumdioxid zugemischt werden.

Typische Zusammensetzungen für einzusetzende Haftschmelzklebstoffe sind solche, die aus zwischen 10 und 80 Gew.%, bevorzugt 20 bis 80 Gew.% und besonders bevorzugt 20 bis 50 Gew.% Polymer, zwischen 1 und 80 Gew.% Weichmacher; zwischen 10 und 80 Gew.%, bevorzugt 15 bis 60 Gew.% Tackifier, ggf. 0.1 bis 5 Gew.% Alterungsschutzmittel und ggf.0 bis 70 Gew.% Füllstoffe hergestellt sind, wobei die Summe der Prozentsätze der Bestandteile stets 100 ist.

Bevorzugt ist, daß der Haftschmelzklebstoff zu 10 bis 50 Gew.% Styrol - Isopren - Styrol Synthesekautschuk, wie es bspw. im Handel unter der Bezeichnung CARIFLEX TR 1107 von der Fa.SHELL erhältlich ist; zwischen 10 und 80 Gew.% eines hydrierten Alkohols, wie er bspw. unter der Bezeichnung ABITOL von der Fa. HERCULES erhältlich ist; zwischen 10 und 80 Gew.% eines Kohlenwasserstoffharzes, bspw. des Harzes HERCURES C von der Fa. HERCULES, zwischen 1 und 40 Gew.% an Estern pflanzlicher Fettsäuren, bspw. MIGLYOL 812 von der Fa. DYNAMIT NOBEL; und ggf. bis zu 5 Gew.% Alterungsschutzmittel, wie Hydrochinon etc. sowie bis zu 70 Gew.% Füllstoffe aufweist.

Bei einer weiteren bevorzugten Ausführungsform der Erfindung weist der Haftschmelzklebstoff zu 10 bis 50 Gew.% eines Polycaprolactons, bspw. CAPA 650 von der Fa.INTEROX; zwischen 10 und 80 Gew.% eines hydrierten Alkohols, bspw. ABITOL von der Fa. HERCULES zwischen 10 und 80 Gew.% eines Kohlenwasserstoffharzes, bspw. HERCURES C von der Fa. HERCULES, zwischen 1 und 40 Gew.% an Estern pflanzlicher Fettsäuren, wie MIGLYOL 812 von der Fa.DYNAMIT NOBEL und ggf. bis zu 5 Gew.% Alterungsschutzmittel, sowie bis zu 70 Gew.% Füllstoffe auf.

Es kann auch bevorzugt sein, daß der Haftschmelzklebstoff zu 10 bis 50 Gew.% Polyethylen-Vinylacetat, wie EVATANE 28-25 von der Fa.ATOCHEM, zwischen 10 und 80 Gew.% eines hydrierten Alkohols bspw. ABITOL von der Fa. HERCULES, zwischen 10 und 80 Gew.% eines Kohlenwasserstoffharzes, bsw. des Harzes HERCURES C von der Fa.HERCULES zwischen 1 und 40 Gew.% an Estern pflanzlicher Fettsäuren, bspw. MIGLYOL 812 von der Fa. DYNAMIT NOBEL und ggf. bis zu 5 Gew.% Alterungsschutzmittel, wie Hydrochinon etc. sowie bis zu 70 Gew.% Füllstoffe aufweist.

Ein geeigneter Haftschmelzklebstoff kann zu 10 bis 50 Gew.% Polyurethan; wie z.B. LUPHEN P 1110 der Fa. BASF zwischen 10 und 80 Gew.% eines hydrierten Alkohols, bspw. ABITOL von der Fa. HERCULES zwischen 10 und 80 Gew.% eines Kohlenwasserstoffharzes, bspw. des Harzes HERCURES C von der Fa.HERCULES; zwischen 1 und 40 Gew.% an Estern pflanzlicher Fettsäuren bspw. MIGLYOL 812 von der Fa. DYNAMIT NOBEL und ggf. bis zu 5 Gew.% Alterungsschutzmittel, sowie bis zu 70 Gew.% Füllstoffe aufweisen.

Es ist auch möglich, daß der Haftschmelzklebstoff zu 10 bis 50 Gew.% Polyamid, wie z.B. EURELON 930 der Fa. SCHERING; zwischen 10 und 80 Gew.% eines hydrierten Alkohols, bspw. ABITOL von der Fa. HERCULES; zwischen 10 und 80 Gew.% eines Kohlenwasserstoffharzes, bspw. des Harzes HERCURES C von der Fa. HERCULES zwischen 1 und 40 Gew.% an Estern pflanzlicher Fettsäuren, bspw. MIGLYOL 812 von der Fa. DYNAMIT NOBEL und ggf. bis zu 5 Gew.% Alterungsschutzmittel, sowie bis zu 70 Gew.% Füllstoffe aufweist.

Es kann auch ein Haftschmelzklebstoff mit 10 bis 50 Gew.% Epoxid, bspw. z.B. EUREPOX 7001 der Fa. SCHERING, zwischen 10 und 80 Gew.% eines hydrierten Alkohols, bspw. ABITOL von der Fa. HERCULES; zwischen 10 und 80 Gew.% eines Kohlenwasserstoffharzes, bspw. des Harzes HERCURES C von der Fa.HERCULES; zwischen 1 und 40 Gew.% an Estern pflanzlicher Fettsäuren, bspw. MIGLYOL 812 von der Fa.DYNAMIT NOBEL und ggf. bis zu 5 Gew.% Alterungsschutzmittel, wie Hydrochinon etc. sowie bis zu 70 Gew.% Füllstoffe verwendet werden.

Ein weiterer für das erfindungsgemäße Verfahren verwendbarer Haftschmelzklebstoff weist zu 10 bis 50 Gew.% Polyisobuten mit zähklebriger kautschukartiger Konsistenz, wie z.B. OPPANOL B 50 der Fa. BASF, zwischen 10 und 80 Gew.% eines hydrierten Alkohols, bspw. ABITOL von der Fa. HERCULES, zwischen 10 und 80 Gew.% eines Kohlenwasserstoffharzes, bspw. des Harzes HERCURES C von der Fa.HERCULES; zwischen 1 und 40 Gew.% an Estern pflanzlicher Fettsäuren, bspw. MIGLYOL 812 von der Fa. DYNAMIT NOBEL; und ggf. bis zu 5 Gew.% Alterungsschutzmittel sowie bis zu 70 Gew.% Füllstoffe auf.

Schließlich ist es bevorzugt, Haftschmelzklebstoffe auf Polyesterbasis, die bspw. zwischen 10 und 80 Gew.% eines hydrierten Alkohols, bspw. ABITOL von der Fa. HERCULES, zwischen 10 und 80 Gew.% eines Kohlenwasserstoffharzes, bspw. des Harzes HERCURES C von der Fa.HERCULES zwischen 1 und 40 Gew.% an Estern pflanzlicher Fettsäuren, bspw. MIGLYOL 812 von der Fa. DYNAMIT NOBEL; und ggf. bis zu 5 Gew.% Alterungsschutzmittel, sowie bis zu 70 Gew.% Füllstoffe aufweisen, einzusetzen.

Nach dem erfindungsgemäßen Verfahren können Vorrichtungen mit einem oder mehrere Stoffdepots, in dem/nen der Stoff mit gegenüber der nicotinaufweisenden Haftschmelzklebstoffschicht erhöhter Konzentration vorliegt, hergestellt werden, wodurch höhere Dosen des Nicotins verarbeitet werden können und damit die Vorrichtung länger im Einsatz bleiben kann, bevor sie ausgewechselt werden muß. Typische Ausgestaltungen finden sich bspw. in der DE-OS 36 29 304.0. Bevorzugte Ausgestaltungen der Erfindung sind in den Unteransprüchen aufgeführt, auf die hiermit ausdrücklich bezug genommen wird.

Die Bildung der Bestandteile der Vorrichtung, die Haftschmelzkleber mit einer Verarbeitungstemperatur zwischen 40 und 80° C aufweisen, kann durch Extrusion, Gießen, Walzenauftrag, Rakelauftrag, Aufsprühen oder ein Druckverfahren erfolgen.

Ein Grenzwert für die Verarbeitbarkeit der Haftschmelzkleber bei vielen dieser Verfahren ist bei einer Viskosität im Bereich von etwa 80 000 Pa.sgegeben.

Falls die mit dem Kleber zu behandelnde Unterlage - eine Komponente der Vorrichtung - durch die Temperatur des heiß aufgetragenen Klebers beschädigt werden könnte - sei es durch Zersetzung, Reaktion oder partielles Schmelzen, kann eine gekühlte Unterlage eingesefzt werden. Die Kühlung kann durch an sich bekannte Verfahren erfolgen, wie durch Einbringung kalter inerter Gase oder das Kontaktieren mit einer Kühlfläche.

Der Haftschmelzkleber kann bspw. in Schichtform oder in einzelnen Bereichen entsprechend einem vorherbestimmten Muster auf die Schutzschicht oder das Abdeckmaterial aufgetragen werden.

Beim Einsatz des leichtfüchtigen und toxischen Nicotins können die nachfolgend genannten besonderen Maßnahmen zur Verarbeitung angezeigt sein:
A. das Arbeiten bei möglichst tiefen Temperaturen
B. die Erhöhung des Außendrucks, um die Verdampfung herabzusetzen,
C. die Sättigung des Dampfraums über der Schmelze mit dem dampfförmigen Stoff
D. das Arbeiten mit entsprechend den Vorgaben kleinstmöglicher Menge an flüchtigem Stoff in der Schmelze.

Aufgrund der Giftigkeit des Nicotins sowie dessen hoher Flüchtigkeit sind dabei in geschlossenen Systemen bzw. gekapselten Vorrichtungen ablaufende Verfahrensvarianten bevorzugt. Selbstverständlich sind diese Maßnahmen, wie bspw. das Arbeiten in einer gekapselten Anlage, durch die dem Fachmann durch den Einsatzzweck der herzustellenden Vorrichtung als auch die stofflichen Gegebenheiten bekannten Gesetzmäßigkeiten limitiert.

Da keine Lösemittel abzudampfen sind, kann die Vorrichtung sogleich nach Auftragen des erwärmten Haftschmelzklebers mit dem Träger- oder Schutzschichtmaterial abgedeckt werden, wodurch eine weitere Verdampfung des Nicotins verhindert werden kann.

Durch das erfindungsgemäße Verfahren kann nun die Verwendung von lösemittelhaltigen haftklebenden Materialien bei der Verarbeitung des leichtflüchtigen Nicotins umgangen werden, was zu erheblicher Erhöhung der Sicherheit der Herstellung, da nun sicher keine toxischen Lösemittelreste in der Arzneimitteldarreichungsform verbleiben, einem stark vereinfachten Verfahren der Auftragung und zu erheblicher Einsparung der Herstellungskosten führt.

Weitere Merkmale und Vorteile der Erfindung werden nachstehend anhand der begleitenden Zeichnung erläutert. Dabei zeigt:
- Fig. 1: einen schematisch dargestellten Schnitt durch ein nach dem erfindungsgemäßen Verfahren hergestelltes Nicotinpflaster mit Nicotindepot;
- Fig. 2: schematisch einen Schnitt durch ein weiteres Nicotinpflaster mit Nicotindepot;
- Fig. 3: einen schematisch dargestellten Schnitt durch ein erfindungsgemäßes Nicotinpflaster ohne Nicotindepot.

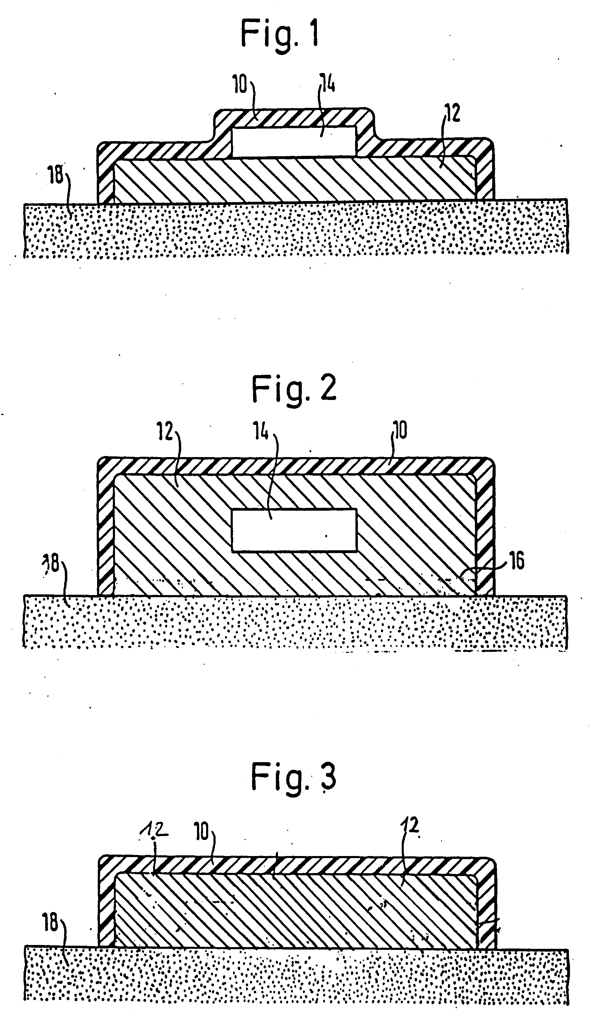

In Fig. 1 ist ein erfindungsgemäß hergestelltes Nicotinpflaster mit einem Nicotindepot 14, einer Haftschmelzkleberschicht 12 sowie einem einer nicotinundurchlässigen Rückschicht 1O, auf der das Nicotindepot 14 aufliegt und das auf die Haut 18 aufgeklebt ist, dargestellt. Es wandert nun kontinuierlich Nicotin mit einer vorher bestimmten Rate durch die Haftschmelzkleberschicht 12 in die Haut 18, wodurch der Nicotingehalt in der Haftschmelzkleberschicht abnimmt. Die Nicotinabnahme wird durch Nachströmen von Nicotin aus dem Nicotindepot 14 kompensiert, so daß über einen vorher bestimmten Zeitraum eine Gleichgewichtskonzentration der Nicotinkonzentration im Haftschmelzkleber 12 herrscht, die für die Abgabe einer konstanten Nicotinmenge an die Haut 18 sorgt.

Dabei ist davon auszugehen, daß das Nicotindepot 14 hochkonzentriert Nicotin aufweisen kann, das beispielsweise an einem inerten Träger oder einem Stützmaterial, wie einem textilen Material, absorbiert sein kann.

In Fig. 2 ist eine weitere Ausführungsform einer erfindungsgemäß hergestellten Vorrichtung dargestellt, bei der ein Nicotindepot 14 allseitig von der Haftschmelzklebermasse 12 umgeben ist.

In Fig. 3 ist eine weitere Ausführungsform eines weiteren erfindungsgemäß hergestellten Nicotinpflasters dargestellt, bei dem auf einer undurchlässigen Rückschicht 1O eine nicotinhaltige Haftschmelzkleberschicht 12 derart aufgebracht ist, daß das Rückschichtmaterial 1O die Haftschmelzklebermasse 12 dreiseitig überdeckt. Mit der freien Haftschmelzkleberfläche wird das Pflaster auf die Haut 18 geklebt, so daß ein ganzflächiger Hautkontakt über die Applikationszeit gewährleistet ist und der Übergang des Nicotins an die Haut stets über eine gleichbleibende Fläche mit einer gleichbleibenden Geschwindigkeit erfolgt, was zu konstanten abgegebenen Nicotin-Dosen führt.

Nachfolgend wird die erfindungsgemäß verbesserte Herstellung eines Nicotinpflasters beschrieben.

Zuerst wird eine Mischung von Nicotin mit Haftschmelzkleber hergestellt. Die Mischung wird sodann auf Haftschmelzkleberverarbeitungstemperatur gebracht und sogleich aus der Schmelze auf ein nicotinundurchlässiges Rückschichtmaterial aufge-bracht. Die weitere Verarbeitung, wie Aufbringen eines abhäsiv ausgerüsteten Schutzschichtmaterials erfolgt in üblicher Weise.

## Patentansprüche

1. Verfahren zur Herstellung einer Vorrichtung zur gesteuerten Abgabe von Nicotin mit einem haftklebenden Nicotinreservoir, gekennzeichnet durch Herstellung einer Mischung aus geschmolzenem Haftschmelzkleber mit einer Verarbeitungstemperatur von 40 bis 80°C und Nicotin und Aufbringen der Mischung auf eine Unterlage.

2. Verfahren nach Anspruch 1, dadurch gekennzeichnet, daß die Verarbeitungstemperatur des Haftschmelzklebers zwischen 40 und 60°C und bevorzugt zwischen 40 und 55°C liegt.

3. Verfahren nach einem der vorangehenden Ansprüche, dadurch gekennzeichnet, daß der Haftschmelzkleber auf der Basis von Styrol-Isopren-Styrol-Blockpolymeren, Polycaprolactonen, Ethylen-Vinylacetat-Copolymeren, Polyurethan, Polyepoxiden, Polyisobuten, Polyvinylethern, ggf. unter Zusatz von Weichmachern, Tackifiern, Füllstoffen, Alterungsschutzmitteln und/oder Thixotropiermitteln hergestellt ist.

4. Verfahren nach einem der vorangehenden Ansprüche, dadurch gekennzeichnet, daß der Haftschmelzkleber aus zwischen 10 und 80 Gew.-%, bevorzugt 20 bis 80 Gew.-% und besonders bevorzugt 20 bis 50 Gew.-% Polymer, zwischen 1 und 80 Gew.-% Weichmacher; zwischen 10 und 80 Gew.-%, bevorzugt 15 bis 60 Gew.-% Tackifier, ggf. 0,1 bis 5 Gew.-% Alterungsschutzmittel und ggf. 0 bis 70 Gew.-% Füllstoffen hergestellt ist, wobei die Summe der Prozentsätze der Bestandteile stets 100 ist.

5. Verfahren nach einem der vorangehenden Ansprüche, gekennzeichnet durch kontinuierliches oder diskontinuierliches Aufbringen nicotinhaltigen geschmolzenen Haftschmelzklebers bei einer Temperatur des Haftschmelzklebers zwischen 40 bis 80°C, bevorzugt zwischen 40 bis 60°C, und besonders bevorzugt zwischen 45 bis 55°C, wobei die Unterlage ein Trägermaterial ist.

6. Verfahren nach einem der vorangehenden Ansprüche, gekennzeichnet durch kontinuierliches oder diskontinuierliches Aufbringen nicotinhaltigen geschmolzenen Haftschmelzklebers bei einer Temperatur des Haftschmelzklebers zwischen 40 bis 80°C, bevorzugt zwischen 40 bis 60°C, und besonders bevorzugt zwischen 45 bis 55°C, wobei die Unterlage ein Schutzschichtmaterial ist.

7. Verfahren zur Herstellung eines Pflasters nach einem der vorangehenden Ansprüche, dadurch gekennzeichnet, daß das Aufbringen des geschmolzenen Haftschmelzklebers mit einer Verarbeitungstemperatur zwischen 40 und 80°C durch Extrusion, Gießen, Walzenauftrag, Rakelauftrag, Aufsprühen oder ein Druckverfahren erfolgt.

8. Verfahren nach einem der vorangehenden Ansprüche, dadurch gekennzeichnet, daß der Nicotin aufweisende Haftschmelzkleber in ein oder mehreren Schichten aufgebracht wird.

9. Verfahren nach einem der Ansprüche 1 bis 5 oder 7 bis 8, dadurch gekennzeichnet, daß eine ablösbare Schutzschicht aufgebracht wird.

10. Verwendung der nach einem der Verfahren nach einem der vorangehenden Ansprüche hergestellten Vorrichtung als Atem-, Kontakt- oder Fraßgift in der Schädlingsbekämpfung.

## Claims

1. Process of producing a device for the controlled delivery of nicotine with an adhesive nicotine reservoir, characterised by producing a mixture formed from molten pressure sensitive adhesive, having a processing temperature of between 40 and 80°C, and nicotine and by applying the mixture to a base.

2. Process according to claim 1, characterised in that the processing temperature of the pressure sensitive adhesive lies between 40 and 60°C and preferably between 40 and 55°C.

3. Process according to one of the preceding claims, characterised in that the pressure sensitive adhesive is produced on the basis of styrene-isoprene-styrene block polymers, polycaprolactones, ethylene-vinylacetate copolymers, polyurethane, polyepoxides, polyisobutene, polyvinyl ethers, possibly with the addition of plasticizers, tackifiers, filler materials, antioxidants and/or thixotropic agents.

4. Process according to one of the preceding claims, characterised in that the pressure sensitive adhesive is produced from between 10 and 80% by wt., preferably 20 to 80% by wt. and especially preferably 20 to 50% by wt., polymer, between 1 and 80% by wt. plasticizer, between 10 and 80% by wt., preferably 15 to 60% by wt., tackifier; possibly 0 1 to 5% by wt. anti-oxidant and possibly 0 to 70% by wt. filler materials, the sum of the percentage of the constituent ingredients always being 100.

5. Process according to one of the preceding claims, characterised by continuously or discontinuously applying nicotine-containing molten pressure sensitive adhesive at a temperature of the pressure sensitive adhesive of between 40 and 80°C, preferably between 40 and 60°C and especially preferably between 45 and 55°C the base being a carrier material.

6. Process according to one of the preceding claims, characterised by continuously or discontinuously applying nicotine-containing molten pressure sensitive adhesive at a temperature of the pressure sensitive adhesive of between 40 and 80°C, preferably between 40 to 60°C and especially preferably between 45 to 55°C, the base being a protective layer material.

7. Process of producing a plaster according to one of the preceding claims, characterised in that the molten pressure sensitive adhesive, having a processing temperature of between 40 and 80°C, is applied by extrusion, moulding, roller application, wiper application, spraying or by a printing process.

8. Process according to one of the preceding claims, characterised in that the nicotine-containing pressure sensitive adhesive is applied in one or a plurality of layers.

9. Process according to one of the claims 1 to 5 or 7 to 8, characterised in that a detachable protective layer is applied.

10. Use of the device, produced according to one of the processes according to one of the preceding claims, as a poison, through inhalation, contact or ingestion, for pest control purposes.

## Revendications

1. Procéde de préparation d'un dispositif à libération retardée de nicotine comportant un réservoir de nicotine autocollant, le procédé étant caractérisé par la préparation d'un mélange de colle adhésive fusible fondue avec une température de traitement de 40 à 80°C et de nicotine et par l'application du mélange sur une base.

2. Procédé selon la revendication 1, caractérisé en ce que la température de traitement de la colle fusible est comprise entre 40 et 60°C, de préférence entre 40 et 55°C.

3. Procédé selon l'une quelconque des revendications précédentes, caractérisé en ce que la colle adhésive fusible est préparée à base de polymères-blocs de styrèneisoprène-styrène, de polycaprolactones, de copolymères d'éthylène et d'acétate de vinyle, de polyuréthane, de polyépoxydes, de polyisobutène, d'éthers de polyvinyle, éventuellement avec addition de plastifiants, d'adhésivants, de charges, d'agents anti-vieillissement et/ou d'agents thixotropes.

4. Procédé selon l'une quelconque des revendications précédentes, caractérisé en ce que la colle adhésive fusible est préparée en utilisant entre 10 et 80 % en poids,de préférence entre 20 et 80 % en poids, mieux encore entre 20 et 50 % en poids de polymère, entre 1 et 80 % en poids de plastifiants, entre 10 et 80% en poids, de préférence entre 15 et 60 % en poids d'adhésivants, éventuellement 0,1 à 5 % en poids d'agents antivieillissement et éventuellement 0 à 70 % en poids de charges, la somme des pourcentages des composants étant toujours de 100.

5. Procédé selon l'une quelconque des revendications précédentes, caractérisé par l'application continue ou discontinue de colle adhésive fusible fondue contenant de la nicotine à une température de la colle comprise entre 40 et 80°C, de préférence entre 40 et 60°C, mieux encore entre 45 et 55°C, la base étant une matière de support.

6. Procédé selon l'une quelconque des revendications précédentes, caractérisé par l'application continue ou discontinue de colle adhésive fusible fondue contenant de la nicotine à une température de la colle comprise entre 40 et 80°C, de préférence entre 40 et 60°C,mieux encore entre 45 et 55°C, la base étant une matière en couche de protection.

7. Procédé de préparation d'un pansement selon l'une quelconque des revendications précédentes, caractérisé en ce que l'application de la colle adhésive fusible fondue dont la température de traitement est comprise entre 40 et 80°C se fait par extrusion, coulée, application au rouleau, application à la lame, pulvérisation ou impression.

8. Procédé selon la revendication 7, caractérisé en ce que la colle adhésive fusible contenant la nicotine est appliquée en une ou plusieurs couches.

9. Procédé selon l'une quelconque des revendications 1 à 5 et 7 à 8, caractérisé en ce que l'on applique une couche de protection détachable.

10. Emploi du dispositif préparé selon l'un des procédés de l'une quelconque desrevendications précédentes comme poison à inhaler, à mettre en contact ou à ingérer dans la lutte contre la vermine.
